# FASCICULE DE BREVET EUROPEEN

(11) **EP 2 557 933 B1**
(45) Date de publication et mention de la délivrance du brevet: **11.10.2017**
(21) Numéro de dépôt: 11720151.7
(22) Date de dépôt: 14.04.2011
(51) Int. Cl.: A61K 47/26, A61K 47/44, A61K 36/28, A61K 36/61, A61K 36/53, A61P 33/00, A01N 65/00, A61K 47/10, A61K 47/24, A61K 9/08

(54) **NOUVELLES FORMULATIONS D'INGRÉDIENT(S) ACTIF(S) D'ORIGINE VÉGÉTALE OU LEURS ANALOGUES SYNTHÉTIQUES OU D'EXTRAIT(S) D'ORIGINE VÉGÉTALE LES CONTENANT, ET DE LÉCITHINE**
NEUARTIGE FORMULIERUNGEN VON AKTIVEN INHALTSSTOFFEN PFLANZLICHER HERKUNFT ODER SYNTHETISCHE ANALOGA DARAUS ODER VON EXTRAKTEN PFLANZLICHER HERKUNFT DAMIT UND VON LECITHIN
NOVEL FORMULATIONS OF ACTIVE INGREDIENT(S) OF PLANT ORIGIN OR SYNTHETIC ANALOGUES THEREOF OR OF EXTRACT(S) OF PLANT ORIGIN CONTAINING SAME, AND OF LECITHIN

(30) Priorité: 16.04.2010 FR 1052903
(43) Date de publication de la demande: 20.02.2013
(73) Titulaire: Xeda International, 13670 Saint Andiol (FR)
(72) Inventeur: SARDO, Alberto, F-13160 Chateaurenard (FR)
(74) Mandataire: Lavoix
(86) Numéro de dépôt international: PCT/FR2011/050861
(87) Numéro de publication internationale: WO 2011/128597

(56) Documents cités:
- WO-A1-99/22703
- DE-A1- 1 617 542
- FR-A1- 2 914 146
- FR-A1- 2 923 355
- JP-A- 2008 239 632

## Description

La présente invention concerne une nouvelle formulation d'agents actifs encapsulés par la lécithine, et leurs utilisations en thérapeutique humaine ou vétérinaire, ou en phytosanitaire.

Les ingrédients actifs d'origine végétale et extraits d'origine végétale les contenant présentent généralement une activité biologique particulièrement attractive.

Ainsi, l'eugénol ou l'huile de girofle qui le contient est connue pour son activité anti-germinative, fongicide ou antibactérienne. Le pyrèthre, ainsi que les pyréthrines qui peuvent en être extraites présentent une activité insecticide remarquable.

Néanmoins, ces agents présentent des difficultés de formulation et d'application, liées notamment à leur faible solubilité. Généralement, il est impossible d'en préparer des solutions aqueuses ; ils nécessitent ainsi l'utilisation de solvants ou d'émulsifiants pour stabiliser les émulsions aqueuses.

Il est donc désirable de mettre à disposition de nouvelles formulations de tels agents, en solution aqueuse.
Ainsi selon un premier objet, la présente invention concerne une composition d'un ou plusieurs ingrédient(s) actif(s) d'origine végétale ou analogue(s) synthétique(s) ou extrait(s) d'origine végétale contenant le(s)dit(s) ingrédient(s), en solution aqueuse, ladite composition comprenant :
- de 5 à 30 % (en poids) d'un ou ingrédient(s) actif(s) d'origine végétale ou analogue(s) synthétique(s) choisi(s) parmi l'eugénol, le thymol, le carvacrol, les pyréthrines, les pyréthroïdes et leurs mélanges, ou extrait(s) d'origine végétale contenant le(s)dit(s) ingrédient(s), tel que l'extrait d'origine végétale est une huile essentielle d'origine végétale, choisie parmi l'huile de girofle, l'huile de thym et les extraits de pyrèthre,
- de 15 à 40 % (en poids) de lécithine,
- de 10 à 55 % (en poids) d'alcool,
- de 10 à 35 % (en poids) d'un ou plusieurs sucre(s),
- eau qsp.
telle que le ou les ingrédient(s) actif(s) d'origine végétale ou analogue(s) synthétique(s) ou extrait(s) d'origine végétale contenant le(s)dit(s) ingrédient(s) est(sont) encapsulé(s) par la lécithine.

Les compositions selon la présente invention sont particulièrement avantageuses en ce que ladite solution est stable, exempte d'émulsifiant.

Selon un aspect particulier, la composition selon l'invention ne nécessite aucun autre ingrédient. Elle peut donc consister essentiellement dans les ingrédients précités.

A titre d'extrait d'origine végétale on peut notamment citer les huiles essentielles telles que l'huile de girofle, ou l'huile de thym ou les extraits de plantes, tels que les extraits de fleurs, les extraits de fleurs de pyrèthre notamment.

A titre d'ingrédient actif contenu dans de tels extraits, on peut ainsi notamment citer l'eugénol, l'isoeugénol, le thymol, le carvacrol, les pyréthrines, et notamment la pyréthrine 1, pyréthrine 2, jasmoline 1, jasmoline 2, cinérine 1, cinérine 2.

On entend par « analogue synthétique » les agents actifs obtenus par synthèse ou hémisynthèse dont la structure est similaire ou mime au moins partiellement la structure d'ingrédients actifs d'origine végétale.

A titre d'analogue synthétique desdits ingrédients actifs, on peut notamment citer les analogues synthétiques des pyréthrines, tels que les pyréthroïdes de synthèse tels que bifenthrine, bioresméthrine, deltaméthrine, dépalléthrine, éthofenprox, fenpropathrine, cyperméthrine, fenvalérate, esfenvalérate, cyfluthrine, alphaméthrine, tralométhrine, fluvalinate, perméthrine, lambda-cyhalothrine, flucythrinate, téfluthrine, tralométhrine, zetacyperméthrine, bétacyfluthrine.

Avantageusement, le(s) ingrédient(s) actif(s) d'origine végétale ou extrait(s) d'origine végétale les contenant, ou analogue(s) synthétiques, hydrophobes peu(ven)t être encapsulé(s) par les compositions selon l'invention.

A titre de lécithine, on peut notamment citer la lécithine naturelle ou modifiée, de préférence la lécithine naturelle.

A titre de sucre, on peut notamment citer le saccharose, le glucose, le fructose, le sucrose et le mannose.

A titre d'alcool, on peut notamment citer les alcools aliphatiques et l'éthanol en particulier.

De façon inattendue, il a été découvert qu'en présence de sucre, il était possible d'obtenir une solubilisation complète de la lécithine et de l'ingrédient actif, dans un mélange d'eau et d'alcool, permettant ainsi un enrobage parfait par micro-encapsulation.

Les compositions selon l'invention permettent l'encapsulation de l'ingrédient actif au sein de la lécithine, par encapsulation lamellaire notamment. Contrairement aux dispersions aqueuses contenant des émulsifiants, les solutions selon l'invention résistent au phénomène de « délavement » par la pluie ou les fluides biologiques.

Les compositions selon l'invention permettent donc une durée d'exposition plus importante de l'ingrédient actif.

Généralement, la composition selon l'invention comprend :
- préférentiellement 10 à 20 % d'ingrédient(s) actif(s) d'origine végétale, extraits d'origine végétale les contenant, ou analogue(s) synthétique(s) ou leurs mélanges,
- de préférence de 20 à 30 % de lécithine,
- de préférence 15 à 30 % d'alcool ;
- de préférence 10 à 20 % d'un ou plusieurs sucres, en solution dans l'eau.

Selon un autre objet, la présente invention concerne également le procédé de préparation d'une composition selon l'invention comprenant:
1) la préparation d'une solution du(es)dit(s) sucre(s) dans l'eau ;
2) l'ajout à ladite solution de l'alcool et du ou des ingrédient(s) actif(s) d'origine végétale ou extrait(s) d'origine végétale les contenant, ou analogue(s) synthétiques,
3) l'ajout de la lécithine.

Généralement, l'ajout de la lécithine est réalisé sous agitation, jusqu'à dissolution complète de la lécithine.

Selon un autre objet, la présente invention concerne également un procédé d'encapsulation d'un ou plusieurs ingrédient(s) actif(s) d'origine végétale ou extrait(s) d'origine végétale les contenant, ou analogue(s) synthétiques, par la lécithine, comprenant la mise en oeuvre du procédé ci-dessus.

Selon un premier mode de réalisation, on peut ainsi citer les compositions comprenant :
- de l'huile de girofle ou de l'eugénol,
- de la lécithine,
- de l'alcool,
- un ou plusieurs sucres,
en solution dans l'eau.

Selon un autre mode de réalisation, la présente invention concerne également les compositions comprenant à titre d'ingrédient actif, un extrait de pyrèthre ou une ou plusieurs pyréthrines naturelles ou leurs analogues synthétiques, en combinaison avec de l'eugénol ou de l'huile de girofle.

Lesdites compositions contiennent alors :
- un extrait de pyrèthre, ou une ou plusieurs pyréthrine(s) naturelles ou pyréthroïde(s) synthétique(s),
- de la lécithine,
- de l'alcool,
- un ou plusieurs sucre(s),
- de l'eau,
en solution.

Les dites compositions peuvent en outre comprendre de l'eugénol ou de l'huile de girofle.

Généralement, les compositions sont telles que le rapport huile de girofle/pyrèthre (poids) est compris entre 1 et 5.

Selon un autre objet, la présente invention concerne une composition pour l'utilisation thérapeutique, humaine ou vétérinaire, la composition selon l'invention comprenant un ou plusieurs ingrédient(s) actif(s) d'origine végétale ou extrait(s) d'origine végétale les contenant, ou analogue(s) synthétiques ayant une activité thérapeutique.

Ledit médicament est destiné à un usage thérapeutique humain ou vétérinaire.

Ainsi, l'eugénol et l'huile de girofle présentent une activité thérapeutique, et notamment bactéricide, fongicide, antiparasitaire. Les compositions selon l'invention les contenant sont ainsi particulièrement adaptées pour le traitement et/ou la prévention de pathologies choisies parmi les parasitoses intestinales, les mycoses, les pathologies bactériennes et/ou les pathologies liées aux organismes unicellulaires telles que les amibes.

Les médicaments selon l'invention comprennent une composition selon l'invention et éventuellement au moins un véhicule pharmaceutiquement acceptable. Ledit véhicule peut être choisi en fonction des applications envisagées. Ainsi, au cas où une application cutanée serait envisagée, elles peuvent être formulées avec un agent de formulation pour administration topique, tel que les véhicules pour la formulation de crème, gel, pommade ou lotion et notamment le beurre de cacao, la cire d'abeille ... De tels véhicules sont connus en soi et à la portée de l'homme du métier.

Les compositions selon l'invention peuvent également être administrées avantageusement par voie orale ; elles seront généralement présentées sous forme de soluté ou solution. Elles pourront éventuellement comprendre des agents édulcorants et/ou arôme, le cas échéant.

La posologie peut varier dans des limites importantes (0,5 milligrammes à 1000 milligrammes de principe actif) en fonction de l'indication thérapeutique et de la voie d'administration, ainsi que de l'âge et du poids du sujet.

Selon un autre objet, la présente invention concerne l'utilisation phytosanitaire d'une composition selon l'invention comprenant un ou plusieurs ingrédient(s) actif(s) d'origine végétale ou extrait(s) d'origine végétale les contenant, ou analogue(s) synthétiques ayant une activité phytosanitaire.

Ainsi, les extraits de pyrèthre, pyréthrines et pyréthroïdes sont des insecticides connus. Les compositions selon l'invention les contenant conviennent particulièrement à un usage insecticide.

D'autre part, les pyréthrines ou leurs analogues synthétiques sont généralement utilisés en combinaison avec le pipéronylbutoxyde à titre d'adjuvant. Néanmoins ce composé présente des effets secondaires controversé et n'est pas d'origine naturelle.

Il a été découvert que les compositions selon l'invention comprenant un ou plusieurs extrait(s) de pyrèthre, pyréthrine(s) et pyréthroïde(s) en combinaison avec l'eugénol ou l'huile de girofle présentent une activité insecticide très intéressante, en comparaison des combinaisons des insecticides précités avec le pipéronylbutoxyde.

Pour l'utilisation à des fins insecticides notamment, lesdites compositions peuvent être soit telles quelles, soit en les ajoutant à l'eau de façon à former une dispersion.

Généralement, ladite dispersion est réalisée en ajoutant ladite composition à de l'eau, de préférence sous agitation vigoureuse.

La présente invention concerne donc également lesdites compositions en dispersion ainsi obtenues, comprenant une composition selon l'invention en dispersion dans l'eau. Généralement, la concentration desdites dispersions est comprise entre 0,5 et 10%.

Selon un autre objet, la présente invention concerne également le procédé de traitement insecticide comprenant l'application des compositions selon l'invention précitées. Le procédé convient particulièrement au traitement de fruits et légumes, au verger généralement, des plants ou semences.

Généralement, les compositions selon l'invention en dispersion dans l'eau conviennent plus particulièrement à l'application aux vergers, sur fruits et/ou légumes. Généralement, cette application peut être réalisée au moyen de gicleurs. Les compositions en émulsions dans l'eau présentent l'avantage de former des particules extrêmement fines, avec un diamètre moyen compris entre 40 et 80 micromètres. Cette faible taille permet ainsi l'usage de gicleurs à buses de petites tailles, permettant ainsi une meilleure application.

Généralement, les compositions telles quelles ou en dispersion dans l'eau peuvent être avantageusement utilisées pour l'application sur grains, céréales ou oléagineux, notamment l'utilisation pour le traitement des silos.

Les exemples suivants sont donnés à titre illustratif et non limitatif de la présente invention.

### Exemple 1 : préparation d'une solution d'eugénol ou huile de girofle

18 g de sucre sont ajoutés à 19 g d'eau, après dissolution complète du sucre, 21 g d'éthanol suivit de 17 g d'huile de girofle sont ajoutés, le mélange est agité et 25 g de lécithine sont ajoutés. Le mélange est agité jusqu'à dissolution complète de la lécithine.

### Exemple 2 : préparation d'une solution de pyréthre et huile de girofle ou eugénol

18 g de sucre sont ajoutés à 20 g d'eau, après dissolution complète du sucre, 21 g d'éthanol suivit de 4 g d'un extrait de pyrèthre et 12 g d'huile de girofle sont ajoutés, le mélange est agité et 25 g de lécithine sont ajoutés. Le mélange est agité jusqu'à dissolution complète de la lécithine.

### Exemple 3 : mise en évidence de l'effet in vivo d'une composition selon l'invention dans le traitement des amibes

La composition de l'exemple 1 a été administrée à un patient humain atteint d'amibes à la dose de 10 g par jour (5 g le matin et 5 g le soir), pour 3 cycles d'une durée de 3 semaines espacés par 2 périodes de 3 semaines.

Les symptômes ont disparu à l'issue du traitement. L'efficacité du traitement a été caractérisée par analyse nanomicroscopique du sang.

### Exemple 4 : préparation d'une composition en dispersion dans l'eau

La composition de l'exemple 2 est ajoutée à de l'eau à une concentration de 3% (poids), sous agitation.

On obtient une dispersion. La taille des particules ainsi obtenues est comprise entre 40 et 80 micromètres.

## Revendications

1. Composition d'un ou plusieurs ingrédient(s) actif(s) d'origine végétale ou analogue(s) synthétique(s) ou extrait(s) d'origine végétale contenant le(s)dit(s) ingrédient(s), en solution aqueuse, ladite composition comprenant :
- de 5 à 30 % (en poids) d'un ou ingrédient(s) actif(s) d'origine végétale ou analogue(s) synthétique(s) choisi(s) parmi l'eugénol, le thymol, le carvacrol, les pyréthrines, les pyréthroïdes et leurs mélanges, ou extrait(s) d'origine végétale contenant le(s)dit(s) ingrédient(s), tel que l'extrait d'origine végétale est une huile essentielle d'origine végétale, choisie parmi l'huile de girofle, l'huile de thym et les extraits de pyrèthre,
- de 15 à 40 % (en poids) de lécithine,
- de 10 à 55 % (en poids) d'alcool,
- de 10 à 35 % (en poids) d'un ou plusieurs sucre(s),
- eau qsp.
telle que le ou les ingrédient(s) actif(s) d'origine végétale ou analogue(s) synthétique(s) ou extrait(s) d'origine végétale contenant le(s)dit(s) ingrédient(s) est(sont) encapsulé(s) par la lécithine.

2. Composition selon la revendication 1 telle que ladite solution est exempte d'émulsifiant.

3. Composition selon la revendication 1 ou 2 consistant essentiellement en :
- de 5 à 30 % (en poids) d'un ou ingrédient(s) actif(s) d'origine végétale ou analogue(s) synthétique(s) ou extrait(s) d'origine végétale contenant le(s)dit(s) ingrédient(s),
- de 15 à 40 % (en poids) de lécithine,
- de 10 à 52 % (en poids) d'alcool,
- de 10 à 35 % (en poids) d'un ou plusieurs sucre(s),
- eau qsp.

4. Composition selon l'une quelconque des revendications précédentes comprenant:
- de l'huile de girofle et/ou de l'eugénol,
- de la lécithine,
- de l'alcool,
- un ou plusieurs sucre(s),
- de l'eau,
en solution.

5. Composition selon l'une quelconque des revendications 1 à 4 comprenant:
- une ou plusieurs pyréthrine(s) naturelles ou pyréthroïde(s) synthétique(s),
- de la lécithine,
- de l'alcool,
- un ou plusieurs sucre(s),
- de l'eau,
en solution.

6. Composition selon la revendication 5 comprenant en outre de l'eugénol ou de l'huile de girofle.

7. Composition selon la revendication 5 ou 6 en dispersion dans l'eau.

8. Médicament comprenant une composition selon l'une quelconque des revendications 1 à 4 comprenant un ou plusieurs ingrédient(s) actif(s) d'origine végétale ou extrait(s) d'origine végétale les contenant, ou analogue(s) synthétiques ayant une activité thérapeutique avec un véhicule pharmaceutiquement acceptable.

9. Composition selon l'une quelconque des revendications 1 à 4 comprenant un ou plusieurs ingrédient(s) actif(s) d'origine végétale ou extrait(s) d'origine végétale les contenant, ou analogue(s) synthétiques ayant une activité thérapeutique, pour le traitement et/ou la prévention chez l'homme ou l'animal de pathologies choisies parmi les parasitoses intestinales, les mycoses, les pathologies bactériennes, les pathologies liées aux organismes unicellulaires.

10. Procédé de traitement insecticide comprenant l'application d'une composition selon l'une quelconque des revendications 1 à 7.

11. Procédé de préparation d'une composition selon l'une quelconque des revendications 1 à 7 comprenant :
1) la préparation d'une solution du(es)dit(s) sucre(s) dans l'eau ;
2) l'ajout à ladite solution de l'alcool et du ou des ingrédient(s) actif(s) d'origine végétale ou extrait(s) d'origine végétale les contenant, ou analogue(s) synthétique(s), et
3) l'ajout de la lécithine.

12. Procédé d'encapsulation d'un ou plusieurs ingrédient(s) actif(s) d'origine végétale ou extrait(s) d'origine végétale les contenant, ou analogue(s) synthétiques, par la lécithine, comprenant le procédé selon la revendication 11.

## Patentansprüche

1. Zusammensetzung eines oder mehrerer Wirkstoffe(s) pflanzlicher Herkunft oder eines synthetischen/synthetischer Analogs/Analoga oder Extrakte(s) pflanzlicher Herkunft den/die Wirkstoff(e) enthaltend, in wässriger Lösung, die Zusammensetzung umfassend:
- von 5 bis 30% (nach Gewicht) eines oder mehrerer Wirkstoffe(s) pflanzlicher Herkunft oder eines synthetischen/synthetischer Analogs/Analoga ausgewählt aus Eugenol, Thymol, Carvacrol, Pyrethrinen, Pyrethroiden und ihren Mischungen, oder Extrakte(n) pflanzlicher Herkunft, die den/die Wirkstoff(e) enthalten, wobei der Extrakt pflanzlicher Herkunft ein ätherisches Öl pflanzlicher Herkunft ist, ausgewählt aus Nelkenöl, Thymianöl und Pyrethrum-Extrakten,
- von 15 bis 40% (nach Gewicht) Lecithin,
- von 10 bis 55% (nach Gewicht) Alkohol,
- von 10 bis 35% (nach Gewicht) eines oder mehrerer Zucker(s),
- Wasser qs.
wobei der oder die Wirkstoff(e) pflanzlicher Herkunft oder synthetische(n) Analog(a) oder Extrakt(e) pflanzlicher Herkunft den/die Wirkstoff(e) enthaltend durch das Lecithin eingekapselt ist/sind.

2. Zusammensetzung gemäß Anspruch 1, wobei die Lösung frei von Emulgator ist.

3. Zusammensetzung gemäß Anspruch 1 oder 2, im Wesentlichen bestehend aus:
- von 5 bis 30% (nach Gewicht) eines oder mehrerer Wirkstoffe(s) pflanzlicher Herkunft oder eines synthetischen/synthetischer Analogs/Analoga oder Extrakte(n) pflanzlicher Herkunft den/die Wirkstoff(e) enthaltend,
- von 15 bis 40% (nach Gewicht) Lecithin,
- von 10 bis 52% (nach Gewicht) Alkohol,
- von 10 bis 35% (nach Gewicht) eines oder mehrerer Zucker(s),
- Wasser qs.

4. Zusammensetzung gemäß einem der vorhergehenden Ansprüche umfassend:
- Nelkenöl und/oder Eugenol,
- Lecithin,
- Alkohol,
- einen oder mehrere Zucker,
- Wasser,
in Lösung.

5. Zusammensetzung gemäß einem der vorhergehenden Ansprüche 1 bis 4 umfassend:
- einen oder mehrere natürliche(n) Pyrethrin(e) oder synthetische(n) Pyrethrin(e),
- Lecithin,
- Alkohol,
- einen oder mehrere Zucker,
- Wasser,
in Lösung.

6. Zusammensetzung gemäß Anspruch 5, ferner umfassend Eugenol oder Nelkenöl.

7. Zusammensetzung gemäß Anspruch 5 oder 6 als eine Dispersion in Wasser.

8. Medikament umfassend eine Zusammensetzung gemäß einem der Ansprüche 1 bis 4, umfassend einen oder mehrere Wirkstoff(e) pflanzlicher Herkunft oder Extrakt(e) pflanzlicher Herkunft diese enthaltend oder synthetische(s) Analog(a) mit therapeutischer Aktivität, mit einem pharmazeutisch akzeptablen Träger.

9. Zusammensetzung gemäß einem der vorhergehenden Ansprüche 1 bis 4 umfassend einen oder mehrere Wirkstoff(e) pflanzlicher Herkunft oder Extrakt(e) pflanzlicher Herkunft diese enthaltend oder synthetische(s) Analog(a) mit therapeutischer Aktivität, für die Behandlung und/oder die Vorbeugung von Erkrankungen ausgewählt aus Darmparasiten, Pilzen, bakteriellen Erkrankungen, Erkrankungen verbunden mit einzelligen Organismen im Menschen oder Tier.

10. Verfahren zur Insektizidbehandlung umfassend die Anwendung einer Zusammensetzung gemäß einem der Ansprüche 1 bis 7.

11. Verfahren zur Herstellung einer Zusammensetzung gemäß einem der Ansprüche 1 bis 7 umfassend:
1) die Herstellung einer Zuckerlösung in Wasser;
2) Zugabe der Alkohollösung und des einen oder der mehreren Wirkstoffe(s) pflanzlicher Herkunft oder Extrakte(s) pflanzlicher Herkunft diese enthaltend oder synthetischen Analogs/Analoga, und
3) Zugabe des Lecithins.

12. Verfahren zum Einkapseln eines oder mehrerer Wirkstoffe(s) pflanzlicher Herkunft oder Extrakte(s) pflanzlicher Herkunft diese enthaltend, oder synthetischen/synthetischer Analogs/Analoga, mit Lecithin, umfassend das Verfahren gemäß Anspruch 11.

## Claims

1. A composition of one or more active ingredients of plant origin or synthetic analogue(s) or extract(s) of plant origin containing said ingredient(s), in aqueous solution, said composition comprising:
- from 5 to 30 % of one or more active ingredients of plant origin or synthetic analogue(s) selected from eugenol, thymol, carvacrol, pyrethrins, pyrethroids and mixtures thereof or extract(s) of plant origin containing said ingredient(s), such that the extract of plant origin is an essential oil selected from clove oil, thyme oil and pyrethrum extracts,
- from 15 to 40 % of lecithin,
- from 10 to 55 of alcohol,
- from 10 to 35% of one or more sugars,
- water,
such that the active ingredient(s) of plant origin or synthetic analogue(s) or extract(s) of plant origin containing said ingredient(s) are encapsulated by the lecithin.

2. The composition according to claim 1 such that said solution is free of emulsifier.

3. The composition according to claim 1 or 2 essentially consisting in:
- from 5 to 30 % of one or more active ingredients of plant origin or synthetic analogue(s) or extract(s) of plant origin containing said ingredient(s),
- from 15 to 40 % of lecithin,
- from 10 to 52 of alcohol,
- from 10 to 35% of one or more sugars,
- water.

4. The composition according to any of the preceding claims comprising:
- clove oil and/or eugenol,
- lecithin,
- alcohol,
- one or more sugars,
- water,
in solution.

5. The composition according to any of claims 1 to 4 comprising:
- one or more natural pyrethrins or synthetic pyrethroids,
- lecithin,
- alcohol,
- one or more sugars,
- water,
in solution.

6. The composition according to claim 5further comprising eugenol or clove oil.

7. A composition comprising a composition according to claim 5 or 6 as a dispersion in water.

8. A drug comprising a composition according to any of claims 1 to 4, comprising one or more active ingredients of plant origin or extract(s) of plant origin containing them, or synthetic analogue(s) having therapeutic activity with a pharmaceutically acceptable carrier.

9. The composition according to any of claims 1 to 4, comprising one or more active ingredients of plant origin or extract(s) of plant origin containing them or synthetic analogue(s) having a therapeutic activity, for treating and/or preventing in humans or animals, pathologies selected from intestinal parastisoses, mycoses, bacterial pathologies, pathologies related to unicellular organisms.

10. An insecticidal treatment method comprising the application of a composition according to any of claims 1 to.

11. A method for preparing a composition according to any of the claims 1 to 7 comprising:
1) preparing a solution of said sugar(s) in water;
2) adding to said solution the alcohol and the active ingredient(s) of plant origin or extract(s) of plant origin containing them, or synthetic analogue(s), and
3) adding lecithin.

12. A method for encapsulating one or more active ingredients of plant origin or extract(s) of vegetable origin containing them, or synthetic analogue(s), by the lecithin, comprising the method according to claim 11.
